# EUROPÄISCHE PATENTSCHRIFT

(11) **EP 2 084 266 B1**
(45) Veröffentlichungstag und Bekanntmachung des Hinweises auf die Patenterteilung: **31.07.2013**
(21) Anmeldenummer: 07819295.2
(22) Anmeldetag: 25.10.2007
(51) Int. Cl.: C12N 5/07

(54) **ZELLKULTURSYSTEM, VERFAHREN ZU SEINER HERSTELLUNG SOWIE SEINE VERWENDUNG IN DER PRÄKLINISCHEN UNTERSUCHUNG**
CELL CULTURE SYSTEM, METHOD FOR THE PRODUCTION THEREOF AND USE IN PRECLINICAL TESTING
SYSTÈME DE CULTURE CELLULAIRE, SON PROCÉDÉ DE PRÉPARATION ET SON UTILISATION EN EXAMEN PRÉCLINIQUE

(30) Priorität: 25.10.2006 DE 102006051283
(43) Veröffentlichungstag der Anmeldung: 05.08.2009
(60) Teilanmeldung: 12186470.6
(73) Patentinhaber: EDI (Experimentelle & Diagnostische Immunologie) GmbH, 72770 Reutlingen (DE)
(72) Erfinder: SCHMOLZ, Manfred, 72810 Gomaringen (DE)
(74) Vertreter: Vossius & Partner
(86) Internationale Anmeldenummer: PCT/EP2007/009243
(87) Internationale Veröffentlichungsnummer: WO 2008/049607

(56) Entgegenhaltungen:
- HALLER DIRK ET AL: "IL-10 producing CD14low monocytes inhibit lymphocyte-dependent activation of intestinal epithelial cells by commensal bacterian" MICROBIOLOGY AND IMMUNOLOGY, Bd. 46, Nr. 3, 2002, Seiten 195-205, XP002467472 ISSN: 0385-5600 in der Anmeldung erwähnt
- LIU ET AL: "Studies of intestinal drug transport using an in silico epithelio-mimetic device" BIOSYSTEMS, NORTH-HOLLAND, AMSTERDAM, NL, Bd. 82, Nr. 2, November 2005 (2005-11), Seiten 154-167, XP005135883 ISSN: 0303-2647
- TINTUT YIN ET AL: "Monocyte/macrophage regulation of vascular calcification in vitro" CIRCULATION, Bd. 105, Nr. 5, 5. Februar 2002 (2002-02-05), Seiten 650-655, XP002467473 ISSN: 0009-7322 in der Anmeldung erwähnt

## Beschreibung

Die vorliegende Erfindung betrifft ein Zellkultursystem sowie die Verwendung des Zellkultursystems zur präklinischen Erprobung von Wirkstoffen.

Das präklinische Wirkstoffscreening bzw. die präklinische Erprobung von Wirkstoffen ist von besonderer Bedeutung für die medizinische Validierung von Substanzen, ehe sie nach erfolgreichem Bestehen dieser präklinischen Erprobungsphase in klinischen Studien am Menschen getestet werden. Ein weiterer Schwerpunkt der präklinischen Erprobung bildet neben der Feststellung einer medizinischen Hauptwirkung der zu testenden Substanzen auch die Abschätzung möglicher Nebenwirkungen, die sich bei einer weiteren Wirkstoffvalidierung in klinischen Studien ergeben könnten.

So ermöglicht beispielsweise die Früherkennung von unerwünschten Nebenwirkungen Kosteneinsparungen. Zudem kann das Risiko für klinische Testpatienten durch eine ausgereifte präklinische Wirkstoffuntersuchung deutlich reduziert werden.

Als präklinische Untersuchungsmodelle kommen insbesondere Versuchstiere in Betracht. Tierversuche haben den Vorteil, dass die zu untersuchenden Wirkstoffe in vivo charakterisiert werden können. Allerdings sind die hieraus gewonnenen Erkenntnisse aufgrund der teilweise gravierenden Unterschiede zwischen Tier und Mensch nur in begrenztem Umfang auch auf den Menschen übertragbar. In der präklinischen Erprobungsphase von Wirkstoffen kommen weiterhin Zellkulturen als Untersuchungsmodelle zum Einsatz. Der Einsatz von Zellkulturen bietet den Vorteil, dass diese relativ einfach durchzuführen sind. Zudem erlauben Zellkulturen hohe Probendurchsatzraten und sehr gut kontrollierbare Testbedingungen. Weiterhin existieren gegen Zellkulturen keine ethischen Bedenken. Nachteilig ist, dass Zellkulturen als in vitro-Untersuchungsmodelle die sich tatsächlich abspielenden zellulären Vorgänge in menschlichen Geweben nur unzureichend simulieren können.

Eine interessante Weiterentwicklung von Zellkulturen stellen die sogenannten Co-Kulturen dar. Solche Co-Kulturen bestehen aus zwei voneinander räumlich separierten Zellkulturen, zwischen denen jedoch ein Stoffaustausch möglich ist. Die eine Zellkultur enthält regelmäßig Zellen bestimmter Gewebetypen, während die andere Zellkultur bestimmte Blutzellen, insbesondere periphere mononukleare Blutzellen (peripheral blood mononuclear cells, PMBC), enthält. Zum präklinischen Testen von Wirkstoffen werden die Zellkulturen in Anwesenheit der zu testenden Wirkstoffe inkubiert. Die in der Co-Kultur ablaufenden Stoffflüsse werden nach der Inkubationssphase untersucht und ausgewertet. Beispielsweise sind derartige Co-Kulturen aus den Artikeln "IL-10 producing CD14low monocytes inhibit lymphocyte-dependent activation of intestinal epithelial cells by commensal bacteria" (Haller D, Microbiol. Immunol. 2002; 46: 195-205) und "Monocyte/Macrophage Regulation of Vascular Calcification In Vitro" (Tintut Y, Circulation 2002, 105: 650-655) bekannt. Nachteilig hierbei ist, dass letztlich auch Co-Kulturen die tatsächlichen Gegebenheiten in Mensch oder Tier nur unzureichend wiedergeben können, insbesondere im Bereich von immunregulativen Vorgängen. Die mit Hilfe solcher Co-Kulturen gewonnenen Erkenntnisse sind daher hinsichtlich einer verlässlichen Beurteilung bzw. Charakterisierung der getesteten Wirkstoffe stets mit einer gewissen Skepsis zu betrachten. Anderseits werden jedoch die Anforderungen an die Qualität eines präklinischen Wirkstoffcreenings wegen der möglichen klinischen Risiken sowie der allgemein immer höher werdenden Entwicklungskosten für Medikamente ständig größer.

Die Erfindung stellt sich somit die Aufgabe, ein in-vitro Untersuchungsmodell zur präklinischen Erprobung von Wirkstoffen bereitzustellen, welches eine gegenüber den aus dem Stand der Technik bekannten Untersuchungsmodellen verbesserte Abbildung der komplexen physiologischen Verhältnisse im menschlichen und/oder tierischen Organismus und insbesondere eine zuverlässigere Charakterisierung der Wirkstoffe mit Hinblick auf ihre klinische Untersuchung ermöglicht.

Diese Aufgabe wird gelöst durch ein Zellkultursystem, insbesondere zur präklinischen Erprobung von Wirkstoffen, umfassend ein erstes und ein zweites Kompartiment, welche über eine für zellulär sezernierte (ausgeschiedene) Stoffe durchlässige Trennschicht zwischen dem ersten und dem zweiten Kompartiment miteinander in Kommunikation stehen, wobei das erste Kompartiment eine syntope Kultur mit Gewebezellen und phagozytierenden Immunzellen und das zweite Kompartiment eine Kultur mit Blutzellen (Blutzellkultur) aufweist.

Unter einer syntopen Kultur im Sinne der vorliegenden Erfindung soll eine Zellkultur verstanden werden, welche in einem Kompartiment mindestens einen Gewebezelltyp und mindestens einen Zelltyp des Immunsystems aufweist.

Unter Vollblut soll im Sinne der vorliegenden Erfindung Blut mit sämtlichen Blutbestandteilen verstanden werden, einschließlich der Blutzellen und des Blutplasmas sowie der darin enthaltenen vorzugsweise biologisch aktiven Faktoren, wie beispielsweise der Gerinnungsfaktoren, Complementproteine etc.

Unter Priming soll im Sinne der vorliegenden Erfindung eine durch Substanzen, insbesondere durch Botenstoffe, ausgelöste Voraktivierung (Präaktivierung) von Zellen verstanden werden.

Durch die Erfindung werden Zellkultursysteme bereitgestellt, die sich aufgrund der Berücksichtigung einer syntopen Kultur mit Gewebezellen und phagozytierenden Immunzellen sowie einer Kultur mit Blutzellen in ihrer zellulären Komplexität deutlich von den bisher bekannten Co-Kultursystemen abheben. Das erfindungsgemäße Zellkultursystem kann insbesondere als syntope Co-Kultur aufgefasst werden. Im Vergleich zu den bekannten Co-Kultursystemen ermöglicht das erfindungsgemäße Zellkultursystem eine wesentlich komplexere und insbesondere differenziertere Kommunikation zwischen den Zellen. Die zwischen den Zellen des Zellkultursystems stattfindenden Stoffflüsse und Regulationsmechanismen erlauben eine deutlich verbesserte Simulation der tatsächlich stattfindenden zellulären Prozesse im menschlichen und/oder tierischen Organismus. Die zellulär ausgeschiedenen Stoffe können insbesondere mit entsprechenden Zielzellen im Zellkultursystem reagieren und können beispielsweise wieder verbraucht werden. Somit können mit besonderem Vorteil unnatürliche Überkonzentrationen im erfindungsgemäßen Zellkultursystem vermieden werden. Weiterhin verändern die Zielzellen ihre eigene Produktion von Signalstoffen unter dem Einfluss der von den anderen Zellen ausgeschiedenen Botenstoffen. Dadurch wird das gesamte regulatorische Netzwerk des erfindungsgemäßen Zellkultursystems auf sehr physiologische Weise modifiziert. Das erfindungsgemäße Zellkultursystem eignet sich in besonderem Maße zur präklinischen Validierung von Wirkstoffen. Hierzu werden zweckmäßigerweise Zellen einer Spezies verwendet, die in einer späteren klinischen Phase mit den zu erprobenden Wirkstoffen behandelt werden soll. Das Zellkultursystem wird zusammen mit den zu erprobendenden Wirkstoffen inkubiert. Die stattfindenden Stoffflüsse und/oder Stoffveränderungen werden vorzugsweise nach der Inkubationsphase erfasst und können insbesondere mit den

Stoffflüssen und/oder Stoffveränderungen eines Zellkultursystems verglichen werden, welches ohne Wirkstoffe inkubiert wird. Die hieraus abgeleiten Daten bzw. Informationen können als Grundlage für eine zuverlässige Charakterisierung der untersuchten Wirkstoffe herangezogen werden. So können insbesondere verbesserte Aussagen bezüglich der medizinischen Wirksamkeit der Wirkstoffe sowie bezüglich möglicher Risiken, insbesondere mit Hinblick auf eine nachfolgende klinische Untersuchung, getroffen werden.

In einer bevorzugten Ausführungsform sind die Gewebezellen des ersten Kompartiments adhärent. Vorzugsweise haften die Gewebezellen auf der Oberfläche der Trennschicht. Die Trennschicht kann mit geeigneten Stoffen vorbeschichtet sein. Bei den Stoffen kann es sich beispielsweise um Proteine, insbesondere um extrazelluläre Matrixproteine, handeln. Beispielsweise kann die Trennschicht mit Kollagen, Laminin, Tenascin etc. beschichtet sein.

Die Gewebezellen des ersten Kompartiments sind zweckmäßigerweise auf der Oberfläche der Trennschicht vorgezüchtet. Erfindungsgemäß ist es insbesondere vorgesehen, dass die Gewebezellen die Trennschicht zumindest teilweise, vorzugsweise vollständig, bedecken. Die Gewebezellen können die Trennschicht insbesondere schichtförmig, vorzugsweise als Monoschicht, bedecken.

In einer besonders bevorzugten Ausführungsform handelt es sich bei den phagozytierenden Immunzellen insbesondere um Monozyten und/oder Makrophagen. Die Monozyten und Makrophagen stellen innerhalb des Immunsystems eine immunregulatorische Schaltzentrale dar. Sie sind insbesondere an entzündlichen Prozessen im menschlichen und/oder tierischen Körper beteiligt. Vorzugsweise handelt es sich bei der syntopen Kultur des ersten Kompartiments zum eine syntope Kultur aus Gewebezellen und phagozytierenden Immunzellen.

Die Immunzellen lagern sich gewöhnlich an die Gewebezellen an, vorzugsweise unter Ausbildung von intermolekularen Adhäsionen. Die Anlagerung der Immunzellen erfolgt insbesondere auf der Basis von an der Zelloberfläche lokalisierten Rezeptoren. Durch die Berücksichtigung der Immunzellen in dem erfindungsgemäßen Zellkultursystem können die in menschlichen und/oder tierischen Körpern ablaufenden entzündlichen Prozesse deutlich besser simuliert werden. Die hieraus abgeleiteten Ergebnisse im Rahmen eines präklinischen Wirkstoffscreenings ermöglichen somit eine zuverlässigere Charakterisierung der mit Hilfe des erfindungsgemäßen Zellkultursystems getesteten Wirkstoffe.

Bei den Gewebezellen der Erfindung handelt es sich vorzugsweise um Zelltypen, welche in entzündlich erkrankenden Geweben, insbesondere in chronisch-entzündlich erkrankenden Geweben, vorkommen. Bei den Geweben kann es sich insbesondere um Organe handeln. Bei den Gewebezellen kann es sich insbesondere um Zellen eines Gewebetyps handeln. In einer weitergehenden Ausführungsform kann die syntope Kultur mehrere Gewebezelltypen enthalten. Dies erhöht in besonderem Maße die Kommunikations- und Regulationsmöglichkeiten zwischen den Zellen des erfindungsgemäßen Zellkultursystems. Auf diese Weise können die physiologischen Verhältnisse im menschlichen und/oder tierischen Körper, insbesondere auf zellulärer Ebene, in besonders wirkungsvoller Weise simuliert werden.

In einer bevorzugten Ausführungsform handelt es sich bei den Gewebezellen um Epithelzellen und/oder um epithelähnliche Zellen. Besonders bevorzugt handelt es sich bei den Gewebezellen um Epidermis-, Bronchial- und/oder Darmepithelzellen.

In einer weiteren Ausführungsform handelt es sich bei den Gewebezellen um Endothelzellen, bevorzugt um Blutgefäßendothelzellen. Weiterhin ist es bevorzugt, dass es sich bei den Gewebezellen um Hautzellen, insbesondere um Keratinozyten, Fibroblasten und/oder Gelenkhautzellen (sogenannte Synoviozyten), und/oder um Chondrozyten handelt. Weiterhin kommen als Gewebezellen neuronale Zellen und/oder Muskelzellen, insbesondere glatte Muskelzellen, in Frage.

Bevorzugt weist das erste Kompartiment eine syntope Kultur mit Muskelzellen, insbesondere glatten Muskelzellen, und Immunzellen auf, wobei die Muskelzellen vorzugsweise auf der Oberseite der Trennschicht aufgebracht sind. Auf der Unterseite der Trennschicht können insbesondere Endothelzellen aufgebracht sein. Auf diese Weise lassen sich die Verhältnisse von natürlichen Blutgefässen simulieren.

In einer weitergehenden Ausführungsform stammen die Zellen des erfindungsgemäßen Zellkultursystems, insbesondere die Zellen der syntopen Kultur (Gewebezellen und phagozytierenden Immunzellen), aus Zelllinien. Die Zelllinien sind vorzugsweise humanen Ursprungs.

In einer weiteren Ausführungsform stammen die Zellen des erfindungsgemäßen Zellkultursystems aus Gewebeproben und/oder aus Proben von Körperflüssigkeiten. Bei den Proben kann es sich insbesondere um Primärisolate handeln, d.h. um Proben, welche menschlichen und/oder tierischen Körpern entnommen sind. Die Gewebeproben und/oder die Proben der Körperflüssigkeiten sind vorzugsweise humanen Ursprungs. Bei den Körperflüssigkeiten kann es sich insbesondere um Blut oder Urin, vorzugsweise um Blut, handeln.

Beispielsweise können die Zellen der syntopen Kultur des ersten Kompartiments aus Gewebeproben stammen, wohingegen die Zellen des zweiten Kompartiments regelmäßig aus Körperflüssigkeiten, vorzugsweise aus Blut, stammen.

Erfindungsgemäß kann es weiterhin vorgesehen sein, dass das Zellkultursystem sowohl Zellen aus Zelllinien als auch Zellen aus Gewebeproben und/oder aus Proben von Körperflüssigkeiten aufweist. Zweckmäßigerweise enthalten die Kompartimente des erfindungsgemäßen Zellkultursystems jeweils entweder Zellen aus Zelllinien oder Zellen aus Gewebeproben und/oder aus Proben von Körperflüssigkeiten.

Bei den kultivierten Blutzellen des zweiten Kompartiments kann es sich um Blutzellen eines Blutzelltyps handeln. Vorzugsweise handelt es sich bei den Blutzellen um Zellen des Immunsystems, insbesondere um Zellen des peripheren Blutes. Beispielsweise kann es sich bei den Blutzellen um periphere mononukleare Blutzellen (peripheral blood mononuclear cells, PMBC) handeln. Weiterhin können die Blutzellen des zweiten Kompartiments mehrere Blutzelltypen, insbesondere Lymphozyten, Monozyten, Makrophagen, Thrombozyten und/oder Erythrozyten, umfassen. Vorzugsweise handelt es sich bei der Kultur des zweiten Kompartiments um eine Kultur des Vollblutes (sogenannte Vollblutkultur). In einer Vollblutkultur liegen gewöhnlich alle im natürlichen Blut vorkommenden Blutzellen kultiviert vor.

In einer weitergehenden Ausführungsform ist das Vollblut humanen Ursprungs. Bevorzugt handelt es sich bei dem Vollblut um Frischblut. In einer besonders bevorzugten Ausführungsform weist das erfindungsgemäße Zellkultursystem ausschließlich Zellen humanen Ursprungs auf. Dadurch ist eine verbesserte Simulation der physiologischen Verhältnisse im menschlichen Körper möglich. Die Zellen des erfindungsgemäßen Zellkultursystems stammen vorzugsweise aus demselben Organismus, insbesondere aus demselben Patienten.

Die Blutzellkultur des zweiten Kompartiments ist vorzugsweise in Überstand und Sediment getrennt. Im Überstand ist gewöhnlich das Blutplasma enthalten. Im Sediment der Vollblutkultur sind insbesondere die Blutzellen, beispielsweise Erythrozyten, Thrombozyten und Leukozyten, enthalten.

In einer besonders bevorzugten Ausführungsform der Erfindung sind die Gewebezellen aktiviert, vorzugsweise entzündlich verändert. Die Gewebezellen befinden sich insbesondere infolge von im Zellkultursystem anwesenden Aktivatoren, insbesondere proentzündlichen Aktivatoren, in einem aktivierten Zustand. Als Aktivatoren kommen beispielsweise Antigene oder Teile davon, insbesondere Epitope, in Betracht. Bei den Aktivatoren kann es sich auch um Superantige handeln. Die Aktivatoren können mikrobiellen, insbesondere bakteriellen, Ursprungs sein. Vorzugsweise handelt es sich bei den Aktivatoren um Bestandteile von bakteriellen Zellwänden. Bei den Aktivatoren kann es sich insbesondere um Glykane, vorzugsweise um Peptidoglykane, beispielsweise um Zymosan, handeln. Weiterhin kommen Lipopolysaccharide als geeignete Aktivatoren in Frage. Bei den Aktivatoren kann es sich weiterhin um Toxine, beispielsweise Endotoxine, handeln. Durch die Aktivierung der Gewebezellen ist eine Simulation von entzündlichen in vivo Prozessen möglich. Beispielsweise können durch die Aktivierung der Gewebezellen die zellulären Vorgänge bei entzündlich verlaufenden Erkrankungen in zufriedenstellender Weise simuliert werden. Bei den mit Hilfe des erfindungsgemäßen Zellkultursystems simulierbaren Erkrankungen kann es sich insbesondere um Osteoarthritis, rheumatoide Arthritis, Morbus Crohn, Colitis ulcerosa und entzündliche Lungenerkrankungen handeln.

Bevorzugt sind die Gewebezellen durch Botenstoffe, insbesondere durch glykosylierte Proteine und/oder Peptide, vorzugsweise durch Zytokine, aktiviert. Bei den Botenstoffen handelt es sich bevorzugt um proinflammatorische Mediatoren, beispielsweise um Interferone, Interleukine und/oder Tumornekrosefaktoren (TNF). So können die Gewebezellen insbesondere durch mindestens einen Botenstoff aus der Gruppe, umfassend Interferon y, Interleukin-1, Interleukin-6 und Tumornekrosefaktor α (TNFα), aktiviert sein. Durch die Aktivierung der Gewebezellen können die zellulären Vorgänge in entzündlich erkrankten Geweben, insbesondere in entzündlich erkrankten Organen, wirkungsvoll nachgeahmt werden.

In einer weitergehenden Ausführungsform handelt es sich bei den im erfindungsgemäßen Zellkultursystem zellulär sezernierten Stoffen allgemein um sogenannte Indikatoren (Indikatorverbindungen) der zellulären Aktivität, insbesondere um Botenstoffe. Vorzugsweise handelt es sich bei den zellulär sezernierten Stoffen um Zytokine. Die zellulär ausgeschiedenen Stoffe können durch die für sie durchlässige Trennschicht in beide Kompartimente des erfindungsgemäßen Zellkultursystems diffundieren und insbesondere mit den dort vorhandenen Zellen reagieren. Die Reaktion kann beispielsweise auf Wechselwirkungen der sezernierten Stoffe mit Oberflächenrezeptoren der im jeweiligen Kompartiment vorhandenen Zellen beruhen. Dies reduziert nicht nur das Auftreten von unnatürlichen Konzentrationen, insbesondere von unnatürlichen Überkonzentrationen, von Stoffen im erfindungsgemäßen Zellkultursystem. Vielmehr können auf diese Weise auch sekundäre Effekte der zu prüfenden Testsubstanzen überhaupt erst in relevanter Weise sichtbar gemacht werden. Dies ist besonders wichtig für die Untersuchung der Dosis-Wirkungs-Beziehungen der zu testenden Wirkstoffe.

In einer weiteren Ausführungsform handelt es sich bei den Wirkstoffen um natürliche und/oder synthetische Wirkstoffe. Weiterhin kann es sich bei den Wirkstoffen um Wirkstoffmetabolite handeln. Die Metabolite werden beispielsweise durch zellulären Verdau der Wirkstoffe, insbesondere mit Hilfe von Hepatozyten, hergestellt. Die für den zellulären Verdau verwendeten Zellen, insbesondere Hepatozyten, können auch Bestandteil des erfindungsgemäßen Zellkultursystems sein. Weiterhin können die Wirkstoffe in einer geeigneten Verabreichungsform, beispielsweise zusammen mit einem pharmazeutisch verträglichen Träger, vorliegen. Die Wirkstoffe können beispielsweise Bestandteile von Cremes und/oder Salben sein.

Die für die zellulär sezernierten Stoffe durchlässige Trennschicht zwischen dem ersten und dem zweiten Kompartiments weist zweckmäßigerweise Öffnungen, insbesondere Poren, mit einem Durchmesser zwischen 0,1 und 5 µm, insbesondere zwischen 0,2 und 0,45 µm, auf. Die Trennschicht kann insbesondere Bestandteil des ersten oder des zweiten Kompartiments sein. Vorzugsweise handelt es sich bei der Trennschicht um den Boden des ersten oder des zweiten Kompartiments. Die Trennschicht kann weiterhin mit dem ersten oder dem zweiten Kompartiment, insbesondere mit dem ersten Kompartiment, einstückig ausgebildet sein. Erfindungsgemäß ist es besonders bevorzugt, dass die Trennschicht als Membran oder Diaphragma ausgebildet ist. Die Kompartimente des erfindungsgemäßen Zellkultursystems können aus verschiedenen Materialien gebildet sein. Als bevorzugte Materialien kommen Kunststoffe, insbesondere Polystyrol oder Polycarbonat, in Frage. Die Kompartimente des erfindungsgemäßen Zellkultursystems sind vorzugsweise als Behältnisse, insbesondere als Näpfe, Kammern oder Vertiefungen (Wells), ausgebildet. Weiterhin können die Kompartimente Teil eines Trägers sein, welcher vorzugsweise mehrere Kompartimente aufweist. So kann es sich bei dem Träger beispielsweise um eine Lochplatte, insbesondere um eine 6-Loch-, 12-Loch-, 24-Loch- oder 96-Lochplatte, handeln. Bei dem Träger kann es sich weiterhin um ein allgemein kommerziell erhältliches Trans-Well-System für Co-Kulturen handeln.

In einer bevorzugten Ausführungsform handelt es sich bei dem ersten Kompartiment um ein oberes Kompartiment und bei dem zweiten Kompartiment um ein unteres Kompartiment des Zellkultursystems.

Die vorliegende Erfindung betrifft weiterhin ein Verfahren zur präklinischen Erprobung von Wirkstoffen unter Verwendung eines Zellkultursystems mit einem ersten und einem zweiten Kompartiment, die über eine für zellulär sezernierte (ausgeschiedene) Stoffe durchlässige Trennschicht zwischen dem ersten und dem zweiten Kompartiment miteinander in Kommunikation stehen, wobei das erste Kompartiment eine syntope Kultur mit Gewebezellen und phagozytierenden Immunzellen und das zweite Kompartiment eine Kultur mit Blutzellen (Blutzellkultur) aufweist, umfassend die Schritte:
- Zugabe der Wirkstoffe zu dem Zellkultursystem,
- Inkubation des Zellkultursystems in Anwesenheit der zugegeben en Wirkstoffe,
- Untersuchung der im Zellkultursystem nachweisbaren Indikatoren zellulärer Aktivität.

In einer bevorzugten Ausführungsform werden die Wirkstoffe zu der syntopen Kultur des ersten Kompartiments hinzugegeben. In manchen Fällen kann es erwünscht sein, den Einfluss von Wirkstoffen auf Gewebe zu untersuchen, wenn die Wirkstoffe vom Blutkreislauf her in die betreffenden Gewebe gelangen. In diesen Fällen ist es erfindungsgemäß bevorzugt, die Wirkstoffe zu der Blutzellkultur des zweiten Kompartiments hinzuzugeben. Auf diese Weise wird der physiologische Blutkreislauf durch die Blutzellkultur des zweiten Kompartiments und das betreffende Gewebe durch die syntope Kultur des ersten Kompartiments simuliert.

In einer besonders bevorzugten Ausführungsform wird eine syntope Kultur aus Gewebezellen und phagozytierenden Immunzellen, vorzugsweise Monozyten und/oder Makrophagen, verwendet.

In einer weiteren Ausführungsform wird das Zellkultursystem, insbesondere die syntope Kultur, vor der Inkubation, vorzugsweise vor der Zugabe der Wirkstoffe, einem sogenannten Priming unterworfen. In einer anderen Ausführungsform kann das Priming auch nach der Zugabe der Wirkstoffe durchgeführt werden. Erfindungsgemäß ist es auch möglich, die Blutzellkultur des zweiten Kompartiments einem Priming zu unterwerfen. Weiterhin können sowohl die syntope Kultur als auch die Blutzellkultur einem Priming unterworfen werden. Das Priming der syntopen Kultur und der Blutzellkultur kann insbesondere mit jeweils unterschiedlichen Stoffen durchgeführt werden. Als Stoffe für das Priming kommen insbesondere Mediatoren oder Aktivatoren, insbesondere entzündliche Aktivatoren, in Betracht. Bezüglich weiterer Einzelheiten und Merkmale hierzu wird auf die bisherige Beschreibung Bezug genommen.

Das Zellkultursystem wird insbesondere während eines Zeitraums von 1 bis 72 Stunden, vorzugsweise von 2 bis 48 Stunden, inkubiert. Die Inkubation des Zellkultursystems erfolgt zweckmäßigerweise bei einer Temperatur zwischen 20°C und 40 °C, insbesondere zwischen 35 °C und 40°C, vorzugsweise bei einer Temperatur von ca. 37 °C. Gegebenenfalls können während der Inkubation des Zellkultursystems weitere Aktivatoren hinzugegeben werden.

In einer weiteren Ausführungsform des erfindungsgemäßen Verfahrens wird die Untersuchung der im Zellkultursystem nachweisbaren Indikatoren zellulärer Aktivität mittels molekularbiologischer Methoden, insbesondere auf transkriptioneller und/oder translationaler Ebene, vorzugsweise auf post-translationaler Ebene, vorgenommen.

In einer weitergehenden Ausführungsform wird zur Untersuchung der Indikatoren zellulärer Aktivität das Kulturmedium, insbesondere in Form einer Kulturflüssigkeit, des ersten und/oder des zweiten Kompartiments untersucht. Vorzugsweise werden von den Zellen des Zellkultursystems sezernierte Stoffe untersucht. Zur Untersuchung der zellulär sezernierten Stoffe werden insbesondere deren Konzentrationen bestimmt. So können beispielsweise zur Untersuchung der zellulär sezernierten Stoffe ELISA-Verfahren (Enzyme linked immunosorbant assay) oder elektrophoretische Verfahren zum Einsatz kommen. Beispielsweise kann eine Gelelektrophorese, insbesondere eine zweidimensionale Polyacrylamid-Gelelektrophorese (2D PAGE), durchgeführt werden. Weiterhin können Arraytechnologien, insbesondere multiplexe Bead-Arrays, verwendet werden. Grundsätzlich kommen alle dem Fachmann geläufigen biologischen Tests in Frage.

Es ist weiterhin bevorzugt, dass zur Untersuchung der Indikatoren zellulärer Aktivität die Zellen des Zellkultursystems untersucht werden. Erfindungsgemäß ist es zweckmäßig, dass die Zellen zur Durchführung der Untersuchung zurückgewonnen werden, vorzugsweise nach der Inkubation des Zellkultursystems.

Bevorzugt werden zur Untersuchung der Indikatoren zellulärer Aktivität zellständige Aktivierungsparameter untersucht. Beispielsweise kann der Calcium-Influx in die Zellen des Zellkultursystems gemessen werden. Weiterhin können die Zellen hinsichtlich ihres cAMP/cGAMP-Spiegels (zyklisches Adenosin-Monophosphat/zyklisches Guanidin-Adenosin-Monophosphat-Spiegel) untersucht werden.

Als bevorzugter zellständiger Aktivierungsparameter kommt weiterhin die Expression von Signaltransducern und/oder Rezeptoren in und/oder auf den Zellen des Zellkultursystems in Betracht. Vorzugsweise wird die Dichte der Signaltransducer und/oder Rezeptoren in und/oder auf den Zellen bestimmt. Als geeignete Untersuchungsmethoden kommen insbesondere Oberflächenmarker-Analysen, beispielsweise histologische Färbungen oder Flow-cytometrische Methoden, in Frage.

Weiterhin können zur Untersuchung von zellständigen Aktivierungsparametern die Zellen des Zellkultursystems auf eine Veränderung, insbesondere Aktivierung oder Inhibierung bzw. Suppression, ihrer Gene untersucht werden. Beispielsweise eignen sich zur Profilerstellung von aktivierten Genen Northern-Blot- und/oder Western-Blot-Analysen. Weiterhin kommen planare Arraytechnologien, insbesondere Gen-Chips, in Betracht. Bevorzugt werden die Gene auf der Basis ihrer Genprodukte untersucht. Vorzugsweise wird von den Zellen des Zellkultursystems gebildete RNA (ribonucleic acid), insbesondere messenger RNA (mRNA), untersucht. Die RNA wird zweckmäßigerweise aus den Zellen isoliert und insbesondere gereinigt. Die Isolierung der RNA aus den Zellen kann beispielsweise durch Extraktion erfolgen. Zur weiteren Untersuchung wird die gebildete RNA vorzugsweise einer Amplifikation, insbesondere einer Polymerase-Kettenreaktion vorzugsweise mit reverser Transkriptase (RT-PCR) unterworfen. Der eigentliche Nachweis der RNA wird gewöhnlich mit Hilfe einer Northern-Blot-Technik vorgenommen.

In einer weiteren Ausführungsform werden zur Untersuchung der Indikatoren zellulärer Aktivität von den Zellen des Zellkultursystems exprimierte Proteine untersucht. So können beispielsweise Expressionsmuster bzw. -profile von den Proteinen erstellt werden. Zur Untersuchung der Proteine können insbesondere massenspektroskopische Methoden herangezogen werden. Bei den Proteinen kann es sich insbesondere um Signaltransducer und/oder Rezeptoren handeln. Der Einsatz von Arraytechnologien ist ebenso möglich. Auch die Charakterisierung apoptotischer Signalwege und Vorgänge im erfindungsgemäßen Zellkultursystem als relevante Endpunkte ist möglich. Die in den vorangegangenen Abschnitten beschriebenen Verfahren sind dem Fachmann hinreichend bekannt, so dass auf eine ausführlichere Darstellung an dieser Stelle verzichtet werden kann.

In einer besonders bevorzugten Ausführungsform des erfindungsgemäßen Verfahrens werden die Indikatoren zellulärer Aktivität im Verhältnis zu den Indikatoren zellulärer Aktivität eines wirkstofffreien Zellkultursystems, d.h. eines Zellkultursystems, welches ohne Wirkstoffe inkubiert wird, untersucht. Die daraus gewonnenen Daten bzw. Informationen erlauben eine nähere Charakterisierung der untersuchten Wirkstoffe. Bezüglich weiterer Einzelheiten und Merkmale wird auf die bisherige Beschreibung verwiesen.

Vorliegend wird ein Kit zur präklinischen Erprobung von Wirkstoffen beschrieben, welcher mindestens ein Kompartiment umfasst, welches eine syntope Kultur mit Gewebezellen und phagozytierende Immunzellen aufweist, insbesondere Monozyten und/oder Makrophagen. Bevorzugt handelt es sich bei der syntopen Kultur um eine syntope Kultur aus Gewebezellen und phagozytierenden Immunzellen. Der Kit kann weiterhin ein Kompartiment mit einem Kulturmedium umfassen, wobei das Kulturmedium vorzugsweise zur Kultivierung von Blutzellen geeignet ist. Das Kulturmedium ist insbesondere eine Kulturflüssigkeit, beispielsweise eine Kulturlösung. Gegebenenfalls kann der Kit ein Blutentnahmebesteck umfassen. Bezüglich weiterer Einzelheiten und Merkmale des Kits wird auf die bisherige Beschreibung verwiesen.

Die vorliegende Erfindung betrifft außerdem die Verwendung des Zellkultursystems zur präklinischen Erprobung von Wirkstoffen, insbesondere zur Untersuchung von Dosis-Wirkungs-Beziehungen der Wirkstoffe. Erfindungsgemäß ist es insbesondere vorgesehen, dass mehrere Zellkultursysteme, insbesondere in Form eines Parallelansatzes, für die präklinische Validierung der Wirkstoffe verwendet werden. Dadurch können beispielsweise mehrere Wirkstoffe parallel und insbesondere vergleichend getestet werden. Ebenso ist es möglich, jedes Zellkultursystem einem unterschiedlichen Priming zu unterwerfen. Erfindungsgemäß kann es weiterhin vorgesehen sein, die in einem Parallelansatz verwendeten Zellkultursysteme auf unterschiedliche Indikatoren zellulärer Aktivität zu untersuchen. Auf diese Weise kann insgesamt eine größere Datenmenge bezüglich der zu untersuchenden Wirkstoffe generiert werden, wodurch die Charakterisierung der Wirkstoffe zusätzlich verbessert werden kann. Bezüglich weiterer Einzelheiten und Merkmale wird auf die bisherige Beschreibung Bezug genommen.

Weitere Merkmale und Einzelheiten der Erfindung ergeben sich aus den nachfolgenden Figurenbeschreibungen. Dabei können die einzelnen Merkmale jeweils für sich alleine oder zu mehreren in Kombination miteinander verwirklicht sein. Die Figuren werden durch Bezugnahme ausdrücklich zum Inhalt der Beschreibung gemacht.

In den Figuren ist schematisch wiedergegeben:
- Figur 1:: Zellkuitursystem,
- Figur 2:: Botenstoff bzw. Mediatorensynthese in klassischen Co-Kultursystemen und einem erfindungsgemäßen Zellkultursystem,
- Figur 3:: Einfluss von Diclofenac auf die Botenstoff bzw. Mediatorensynthese in klassischen Co-Kultursystemen und einem erfindungsgemäßen Zellkultursystem.

### Figurenbeschreibung

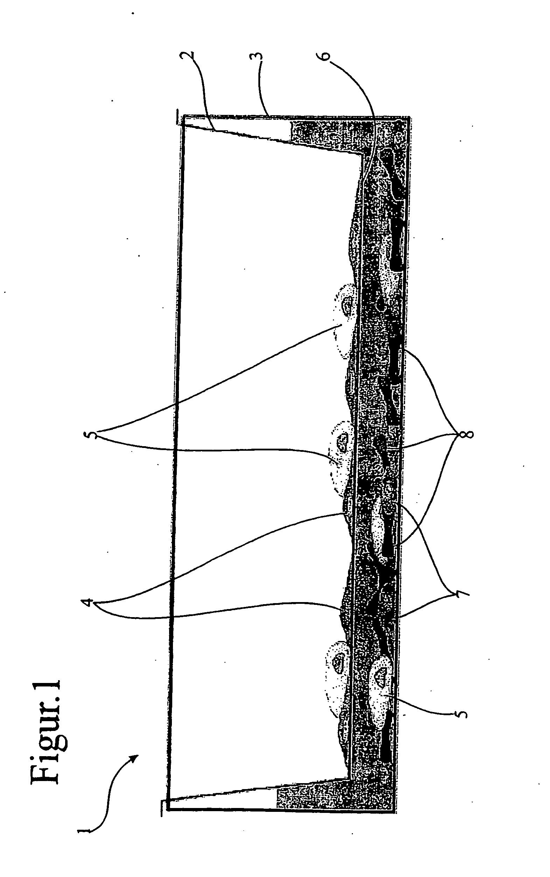

Die Figur 1 zeigt ein erfindungsgemäßes Zellkultursystem 1, welches aus einem als erstes Kompartiment ausgebildeten oberen Behältnis 2 und einem als zweites Kompartiment ausgebildeten unteren Behältnis 3 besteht. Das obere Behältnis 2 weist eine syntope Kultur aus Synoviozyten 4 und Monozyten bzw. Makrophagen 5 auf. Die Synoviozyten 4 sind adhärent und bedecken den als Trennschicht ausgebildeten Boden 6 des oberen Behältnisses 2 schichtförmig. Die Monozyten bzw. Makrophagen 5 liegen auf den Synoviozyten 4 und stehen mit diesen in direktem Kontakt. Die Synoviozyten 4 und die Monozyten bzw. Makrophagen 5 stammen aus Zelllinien humanen Ursprungs. Der Boden 6 des oberen Behältnisses 2 ist für zellulär sezernierte Stoffe durchlässig. Auf diese Weise ist ein Stoffaustausch zwischen dem oberen Behältnis 2 und dem unteren Behältnis 3 möglich. Das untere Behältnis 3 weist eine Vollblutkultur humanen Ursprungs mit Blutzellen 7 und 8 auf. Bei den Blutzellen handelt es sich insbesondere um Monozyten bzw. Makrophagen 5, Lymphozyten 7 und Erythrozyten 8. Die Vollblutkultur ist in Überstand und Sediment getrennt. Die im Zellkultursystem 1 anwesenden Zellen sowie die zwischen den Zellen ablaufenden Stoffflüsse ermöglichen eine verbesserte Simulation der entsprechenden zellulären Vorgänge im menschlichen Organismus. Daher kann das Zellkultursystem 1 in besonders geeigneter Weise für eine präklinische Untersuchung von Wirkstoffen herangezogen werden. Die hieraus gewonnenen Daten bzw. Informationen, insbesondere hinsichtlich einer medizinischen Hauptwirkung, etwaiger Nebenwirkungen sowie einer geeigneten Dosierung der Wirkstoffe, stellen eine verlässliche Beurteilungsgrundlage für die klinische Untersuchung der Wirkstoffe dar.

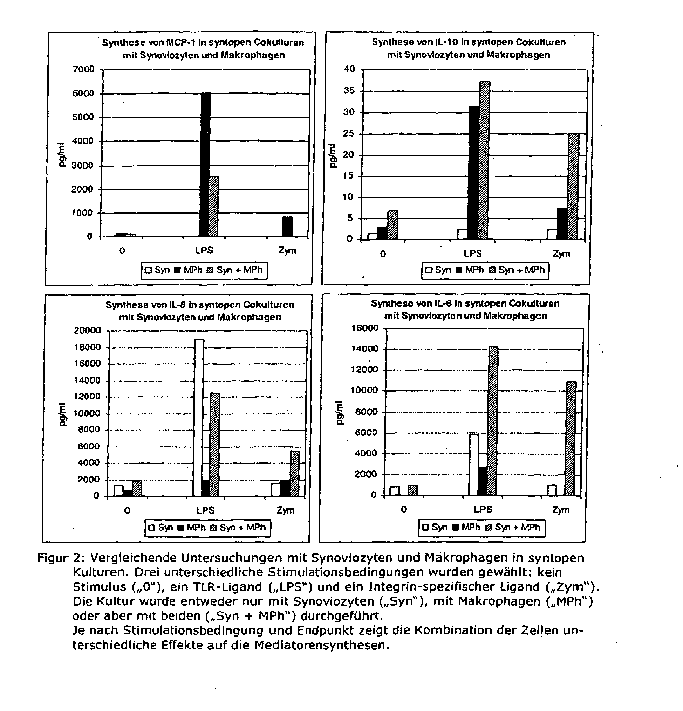

Figur 2 zeigt den Einfluss von verschiedenen Stimulanzien auf die Synthese von Botenstoffen bzw. Mediatoren sowohl bei klassischen Co-Kultursystemen als auch bei einem erfindungsgemäßen Zellkultursystem. Bei den klassischen Co-Kultursystemen handelte es sich um Kultursysteme, welche in ihrem oberen Kompartiment entweder eine Kultur mit Synoviozyten ("Syn") oder eine Kultur mit Makrophagen ("MPh") und in ihrem unteren Kompartiment eine Kultur des Vollblutes (Vollblutkultur) enthielten. Das erfindungsgemäße Zellkultursystem enthielt in seinem oberen Kompartiment eine syntope Kultur aus Synoviozyten und Makrophagen ("Syn + MPh") und in seinem unteren Kompartiment eine Kultur des Vollbluts (Vollblutkultur). Die Kultursysteme wurden drei unterschiedlichen Stimulationsbedingungen ausgesetzt:
- Keine Stimulierung ("0")
- Stimulierung mit einem Lipopolysaccharid ("LPS")
- Stimulierung mit Zymosan ("Zym").

Auf der Ordinate der Figuren 2a bis 2d ist jeweils die Menge des untersuchten synthetisierten Botenstoffes in Picogramm pro Milliliter [pg/ml] wiedergegeben. Auf der Abszisse der Figuren 2a bis 2d sind die Stimulationsbedingungen aufgeführt.

Die Figuren 2a bis 2d zeigen, dass abhängig von den Stimulationsbedingungen, den gemessenen Endpunkten (Synthese von MCP-1 im Falle von Figur 2a, Synthese von IL-10 im Falle von Figur 2b, Synthese von IL-8 im Falle von Figur 2c und Synthese von IL-6 im Falle von Figur 2d) sowie den verwendeten Kultursystemen unterschiedliche Mengen von Botenstoffen synthetisiert wurden.

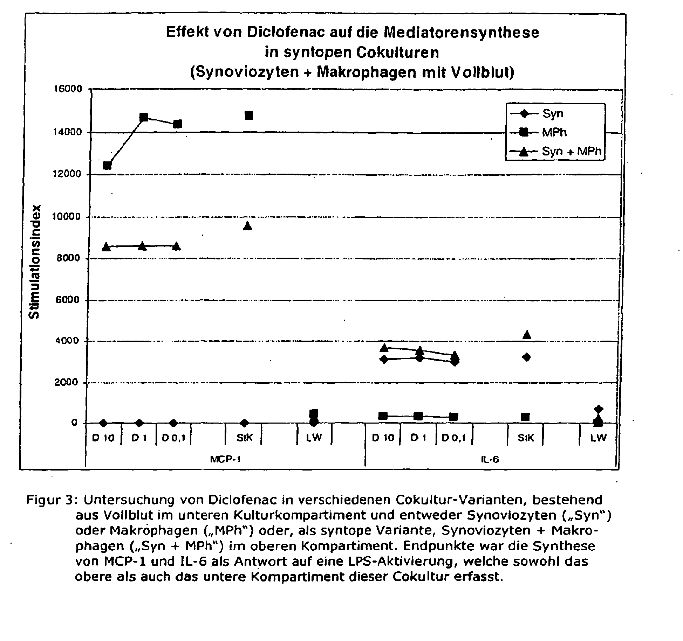

Figur 3 zeigt den Einfluss von Diclofenac (nicht steroidales Analgetikum) auf die Botenstoff- bzw. Mediatorensynthese bei klassischen Co-Kultursystemen und einem erfindungsgemäßen Zellkultursystem. Bezüglich der Merkmale der klassischen Co-Kultursysteme und des erfindungsgemäßen Zellkultursystems wird auf die Figurenbeschreibung zu Figur 2 verwiesen. Als Endpunkte wurde die Synthese von MCP-1 und IL-6 gemessen. Als Stimulationsbedingung wurde eine Aktivierung der Zellen durch ein Lipopolysaccharid gewählt. Auf der Ordinate von Figur 3 ist der sogenannte Stimulationsindex wiedergegeben. Auf der Abszisse von Figur 3 sind die gemessenen Endpunkte aufgeführt.

Die in den Figuren 2 und 3 wiedergegebenen Ergebnisse wurden mittels einer multiplexen Analyse auf der Basis eines sogenannten Bead-Array-Tests mit Hilfe der Luminex(TM)-Technologie durchgeführt. Hierfür wurden Farb-codierte Beads mit spezifischen Antikörper zum Binden der Botenstoffe eingesetzt. Der Gehalt an Botenstoffen wurde mit Hilfe eines zweiten Antikörpers gemessen, welcher fluoreszenzmarkiert war.

## Patentansprüche

1. Zellkultursystem, insbesondere zur präklinischen Erprobung von Wirkstoffen, umfassend ein erstes und ein zweites Kompartiment, welche über eine für zellulär sezernierte Stoffe durchlässige Trennschicht zwischen dem ersten und dem zweiten Kompartiment miteinander in Kommunikation stehen, wobei das erste Kompartiment eine syntope Kultur mit Gewebezellen und phagozytierenden Immunzellen und das zweite Kompartiment eine Kultur mit Blutzellen aufweist.

2. Zellkultursystem nach Anspruch 1 , **dadurch gekennzeichnet, dass** es sich bei den Immunzellen um Monozyten und/oder Makrophagen, handelt.

3. Zellkultursystem nach Anspruch 1 oder 2, **dadurch gekennzeichnet, dass** es sich bei den Gewebezellen um Zellen handelt, welche in entzündlich erkrankenden Geweben vorkommen.

4. Zellkultursystem nach einem der vorhergehenden Ansprüche, **dadurch gekennzeichnet, dass** es sich bei den Gewebezellen um Zellen handelt, die ausgewählt sind aus:
- Epithelzellen und/oder epithelähnliche Zellen,
- Bronchialzellen und/oder Darmepithelzellen,
- Endothelzellen, vorzugsweise Blutgefäßendothelzellen,
- Hautzellen, insbesondere Gelenkhautzellen (Synoviozyten), und/oder Chondrozyten.

5. Zellkultursystem nach einem der vorhergehenden Ansprüche, **dadurch gekennzeichnet, dass** die Zellen des Zellkultursystems aus einem der folgenden stammen:
- Zelllinien, vorzugsweise humanen Ursprungs,
- Gewebeproben und/oder Proben von Körperflüssigkeiten.

6. Zellkultursystem nach einem der vorhergehenden Ansprüche, **dadurch gekennzeichnet, dass** es sich bei der Kultur des zweiten Kompartiments um eine Kultur des Vollbluts (Vollblutkultur), insbesonderevon Frischblut, vorzugsweise humanen Ursprungs, handelt.

7. Zellkultursystem nach einem der vorhergehenden Ansprüche, **dadurch gekennzeichnet, dass** die Gewebezellen aktiviert, insbesondere entzündlich verändert, sind.

8. Zellkultursystem nach einem der vorhergehenden Ansprüche, **dadurch gekennzeichnet, dass** es sich bei den zellulär sezernierten Stoffen um Indikatoren zellulärer Aktivität, insbesondere um Botenstoffe, vorzugsweise um Zytokine, handelt.

9. Zellkultursystem nach einem der vorhergehenden Ansprüche, **dadurch gekennzeichnet, dass** es sich bei den zu erprobenden Wirkstoffen um biologische und/oder synthetische Wirkstoffe handelt.

10. Zellkultursystem nach einem der vorhergehenden Ansprüche, **dadurch gekennzeichnet, dass** die Trennschicht Öffnungen, insbesondere Poren, mit einem Durchmesser zwischen 0,1 und 5 µm, insbesondere zwischen 0,2 und 0,45 µm, aufweist.

11. Zellkultursystem nach einem der vorhergehenden Ansprüche, **dadurch gekennzeichnet, dass** die Kompartimente des Zellkultursystems als Behältnisse, insbesondere als Näpfe, Kammern oder Vertiefungen (Wells), ausgebildet sind.

12. Verfahren zur präklinischen Erprobung von Wirkstoffen unter Verwendung eines Zellkultursystems nach einem der vorhergehenden Ansprüche 1 bis 11, umfassend die Schritte:
- Zugabe von Wirkstoffen zu dem Zellkultursystem,
- Inkubation des Zellkultursystems in Anwesenheit der zugegebenen Wirkstoffe,
- Untersuchung von im Zellkultursystem nachweisbaren Indikatoren zellulärer Aktivität.

13. Verfahren nach Anspruch 12, **dadurch gekennzeichnet, dass** das Zellkultursystem, insbesondere die syntope Kultur, vor der Inkubation, vorzugsweise vor der Zugabe der Wirkstoffe, einem Priming unterworfen wird.

14. Verfahren nach Anspruch 13, **dadurch gekennzeichnet, dass** für das Priming des Zellkultursystems Mediatoren oder Aktivatoren, insbesondere entzündliche Aktivatoren, verwendet werden.

15. Verfahren nach einem der Ansprüche 12 bis 14, **dadurch gekennzeichnet, dass** die Untersuchung der Indikatoren zellulärer Aktivität mittels molekularbiologischer Methoden, insbesondere auf transkriptioneller und/oder translationaler Ebene, vorzugsweise auf post-translationaler Ebene, vorgenommen wird.

16. Verfahren nach einem der Ansprüche 12 bis 15, **dadurch gekennzeichnet, dass** zur Untersuchung der Indikatoren zellulärer Aktivität von den Zellen des Zellkultursystems sezernierte Stoffe und/oder die Zellen des Zellkultursystems untersucht werden.

17. Verfahren nach Anspruch 16, **dadurch gekennzeichnet, dass** im Falle der Untersuchung der Zellen des Zellkultursystems die Zellen des Zellkultursystems zur Durchführung der Untersuchung zurückgewonnen werden.

18. Verfahren nach einem der Ansprüche 12 bis 17, **dadurch gekennzeichnet, dass** zur Untersuchung der Indikatoren zellulärer Aktivität zellständige Aktivierungsparameter, insbesondere die Expression von Signaltransducern und/oder Rezeptoren in und/oder auf den Zellen des Zellkultursystems, untersucht werden.

19. Verfahren nach einem der Ansprüche 12 bis 18, **dadurch gekennzeichnet, dass** die Indikatoren zellulärer Aktivität im Verhältnis zu den Indikatoren zellulärer Aktivität eines wirkstofffreien Zellkultursystems untersucht werden.

20. Verwendung eines Zellkultursystems nach einem der Ansprüche 1 bis 11 zur präklinischen Erprobung von Wirkstoffen, insbesondere zur Untersuchung von Dosis-Wirkungs-Beziehungen der Wirkstoffe.

## Claims

1. A cell culture system, in particular for pre-clinical testing of active substances, comprising a first and a second compartment which are in communication with one another via a separating layer between the first and second compartment, which layer is permeable for secreted substances, wherein the first compartment includes a syntopic culture with tissue cells and phagocytic immune cells and the second compartment includes a culture with blood cells.

2. The cell culture system of claim 1, **characterized in that** the immune cells are monocytes and/or macrophages.

3. The cell culture system of claim 1, **characterized in that** the tissue cells are cells which occur in tissues with inflammatory disorders.

4. The cell culture system of any of the preceding claims, **characterized in that** the tissue cells are cells which are selected from:
- epithelial cells and/or epitheloid cells,
- bronchial and/or intestinal epithelial cells,
- endothelial cells, preferably blood vessel endothelial cells,
- skin cells, in particular synovial intimal cells (synoviocytes) and/or chondrocytes.

5. The cell culture system of any of the preceding claims, **characterized in that** the cells of the cell culture system are derived from one of the following:
- cell lines, preferably cells lines of human origin,
- tissue samples and/or samples of body fluids.

6. The cell culture system of any of the preceding claims, **characterized in that** the culture of the second compartment is a culture of whole blood (whole blood culture), in particular of fresh blood, preferably of human origin.

7. The cell culture system of any of the preceding claims, **characterized in that** the tissue cells are activated, in particular have inflammatory changes.

8. The cell culture system of any of the preceding claims, **characterized in that** the cellularly secreted substances are indicators of cellular activity, in particular messenger substances, preferably cytokines.

9. The cell culture system of any of the preceding claims, **characterized in that** the active substances to be tested are biological and/or synthetic active substances.

10. The cell culture system of any of the preceding claims, **characterized in that** the separating layer has apertures, in particular pores, with a diameter between 0.1 and 5 µm, in particular between 0.2 and 0.45 µm.

11. The cell culture system of any of the preceding claims, **characterized in that** the compartments of the cell culture system are formed as containers, in particular as cups, chambers or wells.

12. A method for pre-clinical testing of active substances using a cell culture system of any of the preceding claims 1 to 11, comprising the steps of:
- adding active substances to the cell culture system,
- incubating the cell culture system in the presence of the added active substances,
- analyzing indicators having cellular activity which are detectable in the cell culture system.

13. The method of claim 12, **characterized in that** the cell culture system, in particular the syntopic culture, is subjected to priming prior to incubation, preferably before the addition of the active substances.

14. The method of claim 13, **characterized in that** mediators or activators, in particular inflammatory activators, are used for priming the cell culture system.

15. The method of any one of claims 12 to 14, **characterized in that** the analysis of the indicators of cellular activity is carried out using molecular biological methods, in particular at the transcriptional and/or translational level, preferably at the post-translational level.

16. The method of any one of claims 12 to 15, **characterized in that**, for the analysis of the indicators of cellular activity, substances secreted by the cells of the cell culture system and/or the cells of the cell culture system are analyzed.

17. The method of claim 16, **characterized in that**, if the cells of the cell culture system are analyzed, the cells of the cell culture system are recovered for carrying out the analysis.

18. The method of any one claims 12 to 17, **characterized in that**, for the analysis of the indicators of cellular activity, cell-associated activating parameters, in particular the expression of signal transducers and/or receptors in and/or on the cells of the cell culture system is analyzed.

19. The method of any one of claims 12 to 18, **characterized in that** the indicators of cellular activity are analyzed in relation to the indicators of cellular activity of a cell culture system which is free of active substances.

20. Use of a cell culture system of any one of claims 1 to 11 for pre-clinical testing of active substances, in particular for testing dose-effect relations of the active substances.

## Revendications

1. Système de culture cellulaire, en particulier pour l'essai préclinique de principes actifs, comprenant un premier et un deuxième compartiment, qui sont en communication l'un avec l'autre par le biais d'une couche de séparation perméable aux substances sécrétées par les cellules, disposée entre le premier et le deuxième compartiment, le premier compartiment présentant une culture syntopique avec des cellules tissulaires et des cellules immunitaires phagocytaires et le deuxième compartiment présentant une culture avec des cellules sanguines.

2. Système de culture cellulaire selon la revendication 1, **caractérisé en ce que** les cellules immunitaires sont des monocytes et/ou des macrophages.

3. Système de culture cellulaire selon la revendication 1 ou 2, **caractérisé en ce que** les cellules tissulaires sont des cellules qui apparaissent dans les tissus atteints d'une maladie inflammatoire.

4. Système de culture cellulaire selon l'une des revendications précédentes, **caractérisé en ce que** les cellules tissulaires sont des cellules qui sont choisies parmi :
- les cellules épithéliales et/ou les cellules épithélioïdes,
- les cellules bronchiques et/ou les cellules épithéliales de l'intestin,
- les cellules endothéliales, de préférence les cellules endothéliales des vaisseaux sanguins,
- les cellules cutanées, en particulier les cellules cutanées articulaires (synoviocytes), et/ou les chondrocytes,

5. Système de culture cellulaire selon l'une des revendications précédentes, **caractérisé en ce que** les cellules du système de culture cellulaire proviennent d'une des origines suivantes :
- de lignées cellulaires, de préférence d'origine humaine,
- d'échantillons de tissus et/ou d'échantillons de fluides corporels.

6. Système de culture cellulaire selon l'une des revendications précédentes, **caractérisé en ce que** la culture du deuxième compartiment est une culture à partir de sang entier (culture de sang entier), en particulier de sang frais, de préférence d'origine humaine.

7. Système de culture cellulaire selon l'une des revendications précédentes, **caractérisé en ce que** les cellules tissulaires sont activées, en particulier modifiées sur le plan inflammatoire.

8. Système de culture cellulaire selon l'une des revendications précédentes, **caractérisé en ce que** les substances sécrétées par les cellules sont des indicateurs de l'activité cellulaire, en particulier des messagers, de préférence des cytokines.

9. Système de culture cellulaire selon l'une des revendications précédentes, **caractérisé en ce que** les principes actifs à étudier sont des principes actifs biologiques et/ou synthétiques.

10. Système de culture cellulaire selon l'une des revendications précédentes, **caractérisé en ce que** la couche de séparation présente des ouvertures, en particulier des pores, ayant un diamètre de 0,1 à 5 µm, en particulier de 0,2 à 0,45 µm.

11. Système de culture cellulaire selon l'une des revendications précédentes, **caractérisé en ce que** les compartiments du système de culture cellulaire sont conçus sous la forme de récipients, en particulier d'alvéoles, de chambres ou de renfoncements (puits).

12. Procédé d'essai préclinique de principes actifs utilisant un système de culture cellulaire selon l'une des revendications précédentes 1 à 11, comprenant les étapes de :
- ajout des principes actifs au système de culture cellulaire,
- incubation du système de culture cellulaire en présence des principes actifs ajoutés,
- étude des indicateurs d'activité cellulaire détectables dans le système de culture cellulaire.

13. Procédé selon la revendication 12, **caractérisé en ce que** le système de culture cellulaire, en particulier la culture syntopique, est soumis à un amorçage avant l'incubation, de préférence avant l'addition des principes actifs.

14. Procédé selon la revendication 13, **caractérisé en ce que** des médiateurs ou des activateurs, en particulier des activateurs inflammatoires, sont utilisés pour l'amorçage du système de culture cellulaire.

15. Procédé selon l'une des revendications 12 à 14, **caractérisé en ce que** l'étude des indicateurs d'activité cellulaire est réalisée au moyen de méthodes de biologie moléculaire, en particulier au niveau de la transcription et/ou de la traduction, de préférence au niveau post-traductionnel.

16. Procédé selon l'une des revendications 12 à 15, **caractérisé en ce que**, pour l'étude des indicateurs d'activité cellulaire des cellules du système de culture cellulaire, des substances sécrétées et/ou les cellules du système de culture cellulaire sont étudiées.

17. Procédé selon la revendication 16, **caractérisé en ce que**, dans le cas de l'étude des cellules du système de culture cellulaire, les cellules du système de culture cellulaire sont récupérées pour la réalisation de l'étude.

18. Procédé selon l'une des revendications 12 à 17, **caractérisé en ce que** pour l'étude des indicateurs d'activité cellulaire, des paramètres d'activation dépendant de la cellule, notamment l'expression de transducteurs de signaux et/ou de récepteurs, sont étudiés dans et/ou sur les cellules du système de culture cellulaire.

19. Procédé selon l'une des revendications 12 à 18, **caractérisé en ce que** les indicateurs d'activité cellulaire sont étudiés par rapport aux indicateurs d'activité cellulaire d'un système de culture cellulaire exempt de principes actifs.

20. Utilisation d'un système de culture cellulaire selon l'une des revendications 1 à 11 pour l'essai préclinique de principes actifs, en particulier l'étude des relations dose-effet des principes actifs.
